# EUROPÄISCHE PATENTANMELDUNG

(11) **EP 1 905 419 A1**
(43) Veröffentlichungstag der Anmeldung: **02.04.2008**
(21) Anmeldenummer: 06019664.9
(22) Anmeldetag: 20.09.2006
(51) Int. Cl.: A61K 8/31, A61K 8/37, A61Q 19/00, C07C 69/24

(54) **Kosmetische Zubereitung und Ölkörper Mischungen**

(71) Anmelder: Cognis IP Management GmbH, 40589 Düsseldorf (DE)
(72) Erfinder: Dierker, Markus, 40597 Düsseldorf (DE); Prinz, Daniela, 41542 Domagen (DE)
(74) Vertreter: Gittinger, Andreas

(57) **Zusammenfassung**

Gegenstand der Erfindung sind **Ölkörper Mischungen,** enthaltend
(A) mindestens einen flüchtigen Kohlenwasserstoff und
(B) mindestens einen Ester ausgewählt aus der Gruppe bestehend aus
(b-1) Estern des **2-Propylheptanols** mit linearen oder verzweigten, gesättigten oder ungesättigten C4-C36-Carbonsäuren - oder C4-C36-Dicarbonsäuren,
(b-2) Estern des **2-Ethylbutanols** mit linearen oder verzweigten, gesättigten oder ungesättigten C4-C36-Carbonsäuren - oder C4-C36-Dicarbonsäuren, und
(b-3) Estern des **2-Butyl-1-octanols** mit linearen oder verzweigten, gesättigten oder ungesättigten C2-C36-Carbonsäuren - oder C2-C36-Dicarbonsäuren

sowie Mischungen daraus sowie ihre Verwendung in kosmetischen Zubereitungen .

## Beschreibung

### Gebiet der Erfindung

Die Erfindung betrifft Ölkörper Mischungen sowie kosmetische Zubereitungen, enthaltend Ölkörper.

### Stand der Technik

Im Bereich kosmetischer Zubereitungen für die Haut- und Haarpflege werden vom Verbraucher eine Vielzahl von Anforderungen gestellt: Abgesehen von den reinigenden und pflegenden Effekten, die den Anwendungszweck bestimmen, wird Wert auf so unterschiedliche Parameter wie höchstmögliche dermatologische Verträglichkeit, gute rückfettende Eigenschaften, elegantes Erscheinungsbild, optimaler sensorischer Eindruck und Lagerstabilität gelegt.

Zubereitungen, die zur Reinigung und Pflege der menschlichen Haut und der Haare eingesetzt werden, enthalten in der Regel neben einer Reihe von oberflächenaktiven Substanzen, vor allem Ölkörper und Wasser. Als Ölkörper/Emollients werden beispielsweise Kohlenwasserstoffe, Esteröle sowie pflanzliche und tierische Öle/Fette/Wachse eingesetzt. Um die höhen Anforderungen des Marktes bezüglich sensorischer Eigenschaften und optimaler dermatologischer Verträglichkeit zu erfüllen, werden kontinuierlich neue Ölkörper und Emulgator-Gemische entwickelt und getestet. Zur Herstellung kosmetischer Zubereitungen werden eine Vielzahl von natürlichen und synthetischen Ölen, beispielsweise Mandel- oder Avocadoöl, Esteröle, Ether, Alkylcarbonate, Kohlenwasserstoffe sowie Silikonöle eingesetzt. Eine wesentliche Aufgabe der Ölkomponenten ist es, neben der pflegenden Wirkung, die in unmittelbarem Zusammenhang mit der Hautfettung steht, dem Konsumenten ein nichtklebriges, möglich rasch eintretendes und lang anhaltendes Gefühl der Hautglätte und -geschmeidigkeit zu vermitteln.

Ölkörper sind wesentlicher Bestandteil der Mehrzahl der kosmetischen Mittel. Obgleich eine Vielzahl von Ölkörpern im Stand der Technik bekannt sind, besteht weiterhin Bedarf nach neuen Ölkörpern, die hinsichtlich Ihrer Verträglichkeit und Formulierbarkeit den bekannten Ölkörpern vergleichbar sind und darüber hinaus vorteilhafte sensorische Eigenschaften aufweisen. Bekannte Silikonöle sind hinsichtlich Ihrer Formulierbarkeit (v.a. hinsichtlich der Emulgierbarkeit) nur eingeschränkt einsetzbar. Es besteht insbesondere ein Bedarf nach Ölkörpern, welche eine den Silikonölen vergleichbare Sensorik auf der Haut erzeugen. Es hat nicht an Versuchen gefehlt, diese "Silikonsensorik" durch silikonfreie Formulierungen zu erreichen, wie z.B. in DE 10361568 beschrieben.

Die Aufgabe der vorliegenden Erfindung hat somit darin bestanden, alternative Ölkörper bereit zu stellen, die in kosmetischen Zubereitungen eingesetzt werden können. Von besonderem Interesse waren Ölkörper, welche in kosmetischen Zubereitungen eingesetzt werden können und diesen - ohne den Zusatz von Silikonölen eine "silikonartige Sensorik" verleihen. Als sensorische Parameter sind hierbei zu nennen: Leichtigkeit, "nicht-fettendes Hautgefühl", Weichheit, Spreitvermögen, Absorption, Verteilbarkeit, Öligkeit. Gleichzeitig war wünschenswert, dass diese Ölkörper eine gegenüber Silikonölen verbesserte Emulgierbarkeit aufweisen.

### Beschreibung der Erfindung

Gegenstand der Erfindung sind **Ölkörper Mischungen,** enthaltend
(A) mindestens einen flüchtigen Kohlenwasserstoff und
(B) mindestens einen Ester ausgewählt aus der Gruppe bestehend aus
   (b-1) Estern des **2-Propylheptanols** mit linearen oder verzweigten, gesättigten oder ungesättigten C4-C36-Carbonsäuren - oder C4-C36-Dicarbonsäuren,
   (b-2) Estern des **2-Ethylbutanols** mit linearen oder verzweigten, gesättigten oder ungesättigten C4-C36-Carbonsäuren - oder C4-C36-Dicarbonsäuren, und
   (b-3) Estern des **2-Butyl-1-octanols** mit linearen oder verzweigten, gesättigten oder ungesättigten C2-C36-Carbonsäuren - oder C2-C36-Dicarbonsäuren sowie Mischungen daraus.

Ein weiterer Gegenstand der Erfindung ist die Verwendung dieser Ölkörper Mischungen in kosmetischen Zubereitungen. In einer Ausführungsform der Erfindung können die Ölkörper Mischungen insbesondere als alleiniger Ölkörper in den kosmetischen Zubereitungen eingesetzt werden.

Die Ölkörper Mischungen eignen sich insbesondere als Silikonersatz in kosmetischen Zubereitungen.

### Ölkörper Mischungen

Als "Ölkörper" im Sinne der Erfindung werden alle wasserunlöslichen Substanzen bezeichnet. Als "wasserunlöslich" werden Substanzen definiert, welche bei Raumtemperatur (23 °C) mit Wasser nicht mischbar sind.

Die erfindungsgemäßen Ölkörper Mischungen können neben den Komponenten (A) und (B) weitere **lipophile Komponenten** enthalten. Diese weiteren lipophilen Komponenten können beispielsweise Öle, Fette, Wachse sowie beliebige Mischungen daraus sein. Diese weiteren lipophilen Komponenten können beispielsweise lipophile Emulgatoren oder liphophile Wirkstoffe, wie beispielsweise Vitamine, sein.

### Weitere lipophile Kompenten

Unter dem Begriff Öle werden wasserunlösliche, bei 30 °C flüssige, organische Verbindungen mit relativ niedrigem Dampfdruck bezeichnet. Das gemeinsame Merkmal der Öle ist nicht ihre übereinstimmende chemische Konstitution, sondere ihre ähnliche physikalische Konsistenz. Unter **Fetten** versteht man Triacylglycerine, also die Dreifachester von Fettsäuren mit Glycerin. Bevorzugt enthalten sie gesättigte, unverzweigte und unsubstituierte Fettsäurereste. Hierbei kann es sich auch um Mischester, also um Dreifachester aus Glycerin mit verschiedenen Fettsäuren handeln. Unter dem Begriff Wachs werden üblicherweise alle natürlichen oder künstlich gewonnenen Stoffe und Stoffgemische mit folgenden Eigenschaften verstanden: sie sind von fester bis brüchig harter Konsistenz, grob bis feinteilig, durchscheinend bis trüb und schmelzen oberhalb von 30°C ohne Zersetzung. Sie sind schon wenig oberhalb des Schmelzpunktes niedrigviskos und nicht fadenziehend und zeigen eine stark temperaturabhängige Konsistenz und Löslichkeit.

In einer bevorzugten Ausführungsform der Erfindung enthalten die Ölkörper Mischungen 60 Gew.-% oder mehr, 70 Gew.-% oder mehr, insbesondere 80 Gew.-% oder mehr, besonders bevorzugt 90 Gew.-% oder mehr, insbesondere 95 Gew.-% oder mehr der Komponenten (A) und (B).

In einer Ausführungsform der Erfindung, enthält die Ölkörper Mischung weniger als 5 Gew.%, insbesondere weniger als 3 Gew.-%, insbesondere weniger als 1 Gew.-% Silikonöle.

In einer Ausführungsform der Erfindung enthält die Ölkörper Mischung keine Silikonöle.

In einer Ausführungsform der Erfindung ist die Menge an (A) größer gleich 50 Gew.-% bezogen auf die Gesamtmenge von (A) und (B), insbesondere größer gleich 60 Gew.%, bevorzugt größer gleich 70 Gew.%. Diese Ölkörper Mischungen zeichnen sich durch Ihre Sensorik aus, insbesondere dadurch, dass sie eine leichte Verteilbarkeit auf der Haut ermöglichen und besonders schnell in die Haut einziehen.

In einer bevorzugten Ausführungsform der Erfindung enthalten die Ölkörper Mischungen 60 Gew.-% oder mehr, 70 Gew.-% oder mehr, insbesondere 80 Gew.-% oder mehr, besonders bevorzugt 90 Gew.-% oder mehr, insbesondere 95 Gew.-% oder mehr der Komponenten (A) und (B) und die Menge an (A) ist größer gleich 50 Gew.-% bezogen auf die Gesamtmenge von (A) und (B), insbesondere größer gleich 60 Gew.-%, bevorzugt größer gleich 70 Gew.-%

Die Erfindung umfasst Ölkörper Mischungen welche mindestens einen flüchtigen Kohlenwasserstoff (A) enthalten und mindestens einen Ester (b-1) des **2-Propylheptanols** mit linearen oder verzweigten, gesättigten oder ungesättigten C4-C36-Carbonsäuren - oder C4-C36-Dicarbonsäuren.

Die Erfindung umfasst Ölkörper Mischungen welche mindestens einen flüchtigen Kohlenwasserstoff (A) enthalten und mindestens einen Ester (b-2) des **2-Ethylbutanols** mit linearen oder verzweigten, gesättigten oder ungesättigten C4-C36-Carbonsäuren - oder C4-C36-Dicarbonsäuren.

Die Erfindung umfasst Ölkörper Mischungen welche mindestens einen flüchtigen Kohlenwasserstoff (A) enthalten und mindestens einen Ester (b-3) des **2-Butyl-1-octanols** mit linearen oder verzweigten, gesättigten oder ungesättigten C2-C36-Carbonsäuren - oder C2-C36-Dicarbonsäuren.

Die Erfindung umfasst ebenso Ölkörper Mischungen, welche mindestens einen flüchtigen Kohlenwasserstoff (A) enthalten und eine Mischungen von jeweils einem oder mehreren Estern (b-1), (4-2) und oder (b-3).

Die erfindungsgemäßen Ölkörper Mischungen lassen sich durch einfaches Mischen der jeweiligen Komponenten (A) und (b-1), (b-2), (b-3) sowie gegebenenfalls weiterer lipophiler Komponenten, gegebenenfalls bei erhöhten Temperaturen herstellen.

### Kosmetische Zubereitungen

Gegenstand der Erfindung sind weiterhin kosmetische Zubereitungen, enthaltend
(A) mindestens einen flüchtigen Kohlenwasserstoff und
(B) mindestens einen Ester ausgewählt aus der Gruppe bestehend aus
   (b-1) Estern des **2-Propylheptanols** mit linearen oder verzweigten, gesättigten oder ungesättigten C4-C36-Carbonsäuren - oder C4-C36-Dicarbonsäuren,
   (b-2) Estern des **2-Ethylbutanols** mit linearen oder verzweigten, gesättigten oder ungesättigten C4-C36-Carbonsäuren - oder C4-C36-Dicarbonsäuren, und
   (b-3) Estern des **2-Butyl-1-octanols** mit linearen oder verzweigten, gesättigten oder ungesättigten C2-C36-Carbonsäuren - oder C2-C36-Dicarbonsäuren sowie Mischungen daraus.

In einer Ausführungsform der Erfindung enthalten die kosmetischen Zubereitungen weniger als 5 Gew.-%, insbesondere weniger als 3 Gew.-%, insbesondere weniger als 1 Gew.-% Silikonöle.

In einer Ausführungsform der Erfindung enthalten die kosmetischen Zubereitungen keine Silikonöle.

Besonders bevorzugt enthalten die kosmetischen Zubereitungen die Komponenten (A) und (B) in solchen Mengen, dass die Menge an (A) größer gleich 50 Gew.-% bezogen auf die Gesamtmenge von (A) und (B), insbesondere größer gleich 60 Gew.-%, bevorzugt größer gleich 70 Gew.-% ist.

In einer bevorzugten Ausführungsform der Erfindung enthält die kosmetische Zubereitung neben (A) und (B) weniger als 40 Gew.-%, insbesondere weniger als 30 Gew.-%, insbesondere weniger als 20 Gew.-%, bevorzugt weniger als 10 Gew.-% weiterer lipophiler Komponenten.

Die erfindungsgemäßen kosmetischen Zubereitungen können ausschließlich aus lipophilen Komponenten bestehen, z.B. in Form von Hautöl, Babyöl, Reinigungsöl. Die erfindungsgemäßen kosmetischen Zubereitungen können aber auch Wasser, Emulgatoren, Tenside sowie weitere übliche kosmetischen Inhaltsstoffe enthalten, wie beispielsweise Konservierungsmittel, biogene Wirkstoffe, UV-Lichtschutzfaktoren, Verdickungsmittel, Überfettungsmittel, Stabilisatoren, Polymere, Antioxidantien, Deodorantien, Filmbildner, Quellmittel, Insektenrepellentien, Hydrotrope, Solubilisatoren, Parfümöle, Farbstoffe etc. Die Mengen der jeweiligen Zusätze richten sich nach der beabsichtigten Verwendung. Die kosmetischen Zubereitungen können neben den Komponenten (A) und (B) noch weitere lipophile Komponenten enthalten. Die Summe der Komponenten (A) und (B) sowie der weiteren lipophilen Komponenten liegt üblicherweise bei 1 bis 90, insbesondere 5 bis 50 Gew%. bezogen auf das Gesamtgewicht der kosmetischen Zubereitung.

Dementsprechend können die erfindungsgemäßen kosmetischen Zubereitungen beispielsweise in folgenden Formen vorliegen: sprühbare Zubereitungen für die Körperpflege (Deosprays, Sonnenschutzsprays usw.), Pflegeprodukte in Gel- oder Cremeform (W/O oder O/W Emulsionen), Wirkstoff-haltige Sprays, Gele oder Cremes, getränkte Pflegetücher oder Pads (Make-up-Entferner, Reinigungstücher usw.) und Ähnliches.

### Komponente (A) Flüchtige Kohlenwasserstoffe

Als Komponente (A) wird mindestens ein flüchtiger Kohlenwasserstoff eingesetzt. Flüchtige Kohlenwasserstoffe sind solche Verbindungen, deren Siedepunkt bei Normaldruck (1013 mbar) kleiner 300 °C ist.

Werden Mischungen aus verschiedenen Kohlenwasserstoffen eingesetzt, so ist maßgebend, dass der Siedepunkt der Hauptfraktion (d.h. 90% des Materials) kleiner 300 °C ist.

In einer bevorzugten Ausführungsform werden als Komponenten (A) unverzweigte Kohlenwasserstoffe eingesetzt.

In einer bevorzugten Ausführungsform der Erfindung werden als flüchtige Kohlenwasserstoffe (A) Kohlenwasserstoffe mit einer C Zahl von größer 6, insbesondere größer 8, bevorzugst größer 10 eingesetzt.

In einer bevorzugten Ausführungsform der Erfindung werden als flüchtige Kohlenwasserstoffe (A) gesättigte und ungesättigte, lineare und verzweigte, cyclische Kohlenwasserstoffe mit einer C Zahl von 6 bis 20, insbesondere mit einer C Zahl von 10 bis 16, eingesetzt.

In einer besonders bevorzugten Ausführungsform der Erfindung werden als flüchtige Kohlenwasserstoffe (A) gesättigte, lineare Kohlenwasserstoffe mit einer C Zahl von 6 bis 20, insbesondere mit einer C Zahl von 10 bis 16, eingesetzt.

Geeignete flüchtige Kohlenwasserstoffe sind beispielsweise n-Decan, n-Undecan, n-Dodecan, n-Tridecan, n-Tetradecan, n-Pentadecan und n-Hexadecan, Isodecan, Isoundecan, Isododecan, Isotridecan, Isotetradecan, Isopentadecan, Isohexadecan, Diethyldecan, Diethyldodecan sowie Mineralöle (sofern sie das Kriterium der Flüchtigkeit erfüllen).

In einer bevorzugten Ausführungsform wird als Komponente (A) mindestens eine Verbindung ausgewählt aus der Gruppe bestehend aus n-Decan, n-Undecan, n-Dodecan, n-Tridecan, n-Tetradecan, n-Pentadecan und n-Hexadecan eingesetzt

Der Begriff "Mineralöle" ist eine Sammelbezeichnung für die aus mineralischen Rohstoffen (Erdöl, Braun- u. Steinkohlen, Holz, Torf) gewonnenen flüssigen Destillationsprodukte, die im Wesentlichen aus Gemischen von gesättigten Kohlenwasserstoffen bestehen. Solche Verbindungen sind als kosmetische Rohstoffe beispielsweise unter den Handelsnamen Halpasole (Fa. Haltermann) erhältlich.

Geeignete flüchtige Kohlenwasserstoffe sind beispielsweise die in WO 03/35707 auf S.6, Z. 25 bis S. 7, Z. 12 beschriebenen Poly-alpha-Olefine, auf die hier ausdrücklich Bezug genommen wird.

Geeignete flüchtige Kohlenwasserstoffe sind beispielsweise die in der internationalen Anmeldung PCT/EP2006/001641 auf S.2 bis 6 beschriebenen Verbindungen sofern sie das Kriterium der Flüchtigkeit erfüllen.

Die flüchtigen Kohlenwasserstoffe (A) können sowohl einzeln als auch in Mischungen untereinander eingesetzt werden.

### Komponente (B) Ester

Die Komponente (B) der erfindungsgemäßen Ölkörper Mischungen bzw. erfindungsgemäßen kosmetischen Zubereitungen enthält mindestens einen Ester ausgewählt aus der Gruppe bestehend aus
(b-1) Estern des **2-Propylheptanols** mit linearen oder verzweigten, gesättigten oder ungesättigten C4-C36-Carbonsäuren - oder C4-C36-Dicarbonsäuren,
(b-2) Estern des **2-Ethylbutanols** mit linearen oder verzweigten, gesättigten oder ungesättigten C4-C36-Carbonsäuren - oder C4-C36-Dicarbonsäuren, und
(b-3) Estern des **2-Butyl-1-octanols** mit linearen oder verzweigten, gesättigten oder ungesättigten C2-C36-Carbonsäuren - oder C2-C36-Dicarbonsäuren sowie Mischungen daraus.

Der Begriff "CX Carbonsäuren" umfasst Carbonsäuren mit einer Gesamtkohlenstoff Anzahl von X, also z.B. "C8 Carbonsäuren" umfasst alle Carbonsäuren, welche eine Gesamt Kohlenstoff Zahl von 8 haben, wie beispielsweise n-Octansäure, Isooctansäuren oder Methylheptansäuren. Entsprechend umfasst der Begriff "CX Dicarbonsäuren" alle Säuren mit 2 Carboxygruppen, welche eine Gesamt C Zahl von X aufweisen, so umfasst z.B. "C4 Dicarbonsäure" u.a. Butandisäure (Bernsteinsäure) sowie Maleinsäure und Fumarsäure.

Der Begriff "Carbonsäure" bezeichnet im Rahmen der vorliegenden Erfindung "Monocarbonsäuren".

Alle erfindungemäß als Komponente (B) einsetzbaren Ester (b-1), (b-2) sowie (b-3) sind können als Säurekomponente

Erfindungsgemäß sind Ester des 2-Propylheptanols (b-1), des 2-Ethylbutanols (b-2) und/oder des 2-Butyl-1-octanols (b-3) mit beispielsweise (in Klammern Trivialnamen der Säuren) n-Butansäure (Buttersäure), 2-Methylpropansäure (Isobuttersäure), Pentansäure (Valeriansäure), i-Pentansäure, wie z.B. 2,2-Dimethylpropansäure (Pivalinsäure, Neopentansäure) und 3-Methylbutansäure (iso-Pentansäure, iso-Valeriansäure), Hexansäure (Capronsäure), Heptansäure, Octansäure (Caprylsäure), i-Octahsäure wie z.B. insbesondere 2-Ethylbutyl-2-ethylhexansäureester, aber auch 2-Ethylbutyl-3-ethylhexansäureester, 2-Ethylbutyl-4-ethylhexansäureester, 2-Ethylbutyl-5-ethylhexansäureester sowie technische Gemische von verzweigten Octansäuren, wie sie beispielsweise unter dem Handelsnamen Cekanoic® C8 von der Fa. Exxon vertrieben werden. Nonansäure (Pelargonsäure, Nonylsäure), Decansäure (Caprinsäure), i-Decansäuren, wie z.B. Trimethylheptansäure (Neodecansäure, Isodecansäure) sowie technische Gemische verzweigter Decansäuren, wie sich beispielsweise unter dem Handelsnamen Cekanoic® C10 von der Fa. Exxon vertrieben werden, Undecansäure, Undecensäure, Dodecansäure (Laurinsäure), Tridecansäure, Tetradecansäure (Myristinsäure), Pentadecansäure, Hexadecansäure (Palmitinsäure), Heptadecansäure (Margarinsäure), Octadecansäure (Stearinsäure), Nonadecansäure, Eicosansäure, Docosansäure, Tetracosansäure, Hexacosanäure, Dimerfettsäuren (C36, wie beispielsweise unter dem Handelsnamen "Empol 1062" von der Fa. Cognis erhältlich) Talkfettsäuren, Kokosfettsäuren, Palmfettsäuren, Rizinolsäure, Ölsäure, Linolsäure, Linolensäure, Isostearinsäure, Isooctansäure, Isononansäure, Isodecansäure, 2-Ethylhexansäure, 2-Propylheptansäure, 2-Butyloctansäure, 2-Butyldecansäure, 2-Hexyloctansäure, 2-Hexyldecansäure, 2-Hexyldodecansäure, 2-Oetyldecansäure, oder Dicarbonsäuren wie z.B. Fumarsäure, Maleinsäure, Adipinsäure, Pimelinsäure, Korksäure, Azelainsäure, Sebazinsäure. Geeignet sind auch Ester des 2-Butyl-1-octanols mit Cekanoic®C8 (Isooctansäure), Cekanoic®C9 (Isononansäure: 3,5,5-Trimethylhexansäure und 2,5,5-Trimethylhexansäure) und Cekanoic®C10 (Isodecansäure) von der Fa. Exxon Mobile, die Carbonsäure-Isomerengemische darstellen.

### Komponenten (b-1) Ester von 2-Propylheptanol

Erfindungsgemäß als Komponenten (B) einsetzbar sind (b-1) Ester des **2-Propylheptanols** mit linearen oder verzweigten, gesättigten oder ungesättigten C4-C36-Carbonsäuren - oder C4-C36-Dicarbonsäuren.

Diese Verbindungen sind in der internationalen Anmeldung PCT/EP2006/002148 beschrieben, auf die hier ausdrücklich Bezug genommen wird.

In einer bevorzugten Ausführungsform der Erfindung werden Ester des 2-Propylheptanols verwendet deren Gesamt C Zahl kleiner gleich 24, bevorzugt kleiner gleich 22 beträgt.

Erfindungsgemäß bevorzugt sind Ester des 2-Propylheptanols mit Carbonsäuren, die ausgewählt sind aus den C4 bis C30, insbesondere C6 bis C24, insbesondere C6 bis C22, insbesondere C6 bis C18, insbesondere C8 bis C18 bevorzugt C8 bis C16, vorzugsweise C8 bis C12, insbesondere C6 bis C10 Carbonsäuren oder den entsprechenden Dicarbonsäuren.

Erfindungsgemäß eignen sich Ester des 2-Propylheptanols mit C4 bis C36, C5 bis C30, C6 bis C26, C7 bis C24, C8 bis C22, C9 bis C20, C10 bis C18, C 11 bis C17, C11 bis C16, C12 bis C15, C13 bis C14 Carbonsäuren oder den entsprechenden Dicarbonsäuren.

In einer besonders bevorzugten Ausführungsform der Erfindung werden Estern des 2-Propylheptanols mit Carbonsäuren, die ausgewählt sind aus den C6 bis C12 Carbonsäure sowie Estern des 2-Propylheptanols mit Dicarbonsäuren, die ausgewählt sind aus den C6 bis C12 Dicarbonsäuren, eingesetzt.

Estern des 2-Propylheptanols mit gesättigten Carbonsäuren sind erfindungsgemäß bevorzugt. Ester des 2-Propylheptanols mit gesättigten Dicarbonsäuren sind erfindungsgemäß bevorzugt. Ester des 2-Propylheptanols mit linearen, unverzweigten Carbonsäuren sind erfindungsgemäß bevorzugt. Estern des 2-Propylheptanols mit linearen, unverzweigten Dicarbonsäuren sind erfindungsgemäß bevorzugt.

Als Ester des 2-Propylheptanols können Ester mit linearen und/oder verzweigten Carbonsäuren eingesetzt werden. Geeignet sind Ester des 2-Propylheptanols mit verzweigten Carbonsäuren: Unter dem Begriff "i-Säure" mit X C-Atomen im Sinne der Erfindung sind alle verzweigten Carbonsäuren zu verstehen, welche in Summe X C-Atome enthalten. So z.B. Methyl-, Ethyl- oder Propylverzweigte, gegebenenfalls mehrfachverzweigte Carbonsäuren. In einer besonderen Ausführungsform wird die Untergruppe der - gegebenenfalls mehrfach - methylverzweigten Carbonsäuren eingesetzt (=Iso-Säuren).

Bevorzugt sind die folgenden Ester 2-Propylheptyl-n-hexansäureester, 2-Propylheptyl-n-heptansäureester, 2-Propyl-heptyl-n-octansäureester, 2-Propyl-heptyl-n-nonansäureester, 2-Propylheptyl-n-decansäureester, 2-Propylheptyl-n-undecansäureester, 2-Propylheptyl-n-dodecansäure-ester.

Besonders bevorzugt sind die folgenden Ester: 2-Propylheptyl-n-hexansäureester, 2-Propylheptyl-n-octansäureester, 2-Propylheptyl-n-decansäureester, 2-Propylheptyl-n-dodecansäureester.

Bevorzugt sind die folgenden Ester 2-Propylheptyl-n-hexansäureester, 2-Propylheptyl-i-hexansäureester, 2-Propylheptyl-n-heptansäureester, 2-Propylheptyl-i-heptansäureester, 2-Propylheptyl-n-octansäureester, 2-Propyl-heptyl-i-octansäureester, 2-Propylheptyl-n-nonansäureester, 2-Propylheptyl-i-nonansäureester, 2-Propylheptyl-n-decansäureester, 2-Propylheptyl-i-decansäureester, 2-Propylheptyl-n-undecansäureester, 2-Propylheptyl-i-undecansäureester, 2-Propylheptyl-n-dodecansäureester, 2-Propylheptyl-i-dodecansäureester.

Besonders bevorzugt sind die folgenden Ester 2-Propylheptyl-n-hexansäureester, 2-Propylheptyl-i-hexansäureester, 2-Propylheptyl-n-octansäureester, 2-Propylheptyl-i-octansäureester, 2-Propylheptyl-n-decansäureester, 2-Propylheptyl-i-decansäureester, 2-Propylheptyl-n-dodecansäureester, 2-Propylheptyl-i-dodecansäureester.

Der Begriff "Ester von 2-Propylheptanol mit Dicarbonsäuren" umfasst sowohl Diester der Dicarbonsäuren mit 2-Propylheptanol, also beispielsweise Di-2-Propylheptyl-n-octandisäurediester als auch Monoester, wie z.B. 2-Propylheptyl-n-octandisäuremonoester als auch gemischte Ester, in denen eine Säuregruppe der Dicarbonsäure mit 2-Propylheptanol verestert ist und die zweite Säuregruppe der Dicarbonsäure mit einem weiteren Alkohol verestert ist. In einer Ausführungsform der Erfindung werden gemischte Ester von Dicarbonsäuren und 2-Propylheptanol und einem weiteren Alkohol, ausgewählt aus der Gruppe bestehend aus 3-Methyl-2-propylhexanol, 4-Methyl-2-propylhexanol und 5-Methyl-2-propylhexanol eingesetzt. In einer bevorzugten Ausführungsform der Erfindung werden die gemischten Ester erhalten durch Umsetzung der entsprechenden Dicarbonsäure mit einem Gemisch aus 2-Propylheptanol, 3-Methyl-2-propylhexanol, 4-Methyl-2-propylhexanol und 5-Methyl-2-propylhexanol. In einer weiteren Ausführungsform werden eingesetzt gemischte Ester von Dicarbonsäuren und 2-Propylheptanol und einem weiteren Alkohol der allgemeinen Formel R-OH, wobei R für einen linearen oder verzweigten, gesättigten oder ungesättigten Alkylrest mit 1 bis 12 C-Atomen steht.

In einer weiteren Ausführungsform werden eingesetzt gemischte Ester von Dicarbonsäuren und 2-Propylheptanol und einem weiteren Alkohol der allgemeinen Formel R-OH, wobei R für einen gesättigten, linearen oder verzweigten, Alkylrest mit 1 bis 12 C-Atomen steht.

In einer bevorzugten Ausführungsform werden verwendet gemischte Ester von Dicarbonsäuren und 2-Propylheptanol und einem weiteren Alkohol, wobei der weitere Alkohol ausgewählt ist aus der Gruppe bestehend aus Methanol, Ethanol, Propanol, Isopropanol Butanol, Isobutanol, Pentanol, Hexanol, Isohexanol, Octanol, Decanol oder Dodecanol.

In einer bevorzugten Ausführungsform der Erfindung werden als Ester von 2-Propylheptanol mit C4 bis C36 Dicarbonsäuren Diester und gemischte Ester verwendet.

Als Diester der Dicarbonsäuren von 2-Propylheptanol seien genannt Di-2-Propylheptyl-n-butandisäurediester, Di-2-Propylheptyl-i-butandisäurediester, Di-2-Propylheptyl-n-pentandisäurediester, Di-2-Propylheptyl-i-pentandisäurediester, Di-2-Propylheptyl-n-hexandisäurediester, Di-2-Propylheptyl-i-hexandisäurediester, Di-2-Propylheptyl-n-heptandisäurediester, Di-2-Propylheptyl-i-heptandi-säurediester, Di-2-Propylheptyl-n-octandisäurediester, Di-2-Propylheptyl-i-octandisäurediester, Di-2-Propylheptyl-n-nonandisäurediester, Di-2-Propylheptyl-i-nonandisäurediester, Di-2-Propylheptyl-n-decandisäurediester, Di-2-Propylheptyl-i-decandisäurediester, Di-2-Propylheptyl-n-undecandi-säurediester, Di-2-Propylheptyl-i-undecandisäurediester, Di-2-Propylheptyl-n-undecendisäurediester, Di-2-Propylheptyl-i-undecendisäurediester, Di-2-Propylheptyl-n-dodecandisäurediester, Di-2-Propylheptyl-i-dodecandisäurediester.

Als gemischte Ester von Dicarbonsäuren von 2-Propylheptanol und Methanol seien genannt 2-Propylheptyl-methyl-n-butandisäurediester, 2-Propylheptyl-methyl-i-butandisäurediester, 2-Propylheptyl-methyl-n-pentandisäurediester, 2-Propylheptyl-methyl-i-pentandisäurediester, 2-Propylheptyl-methyl-n-hexandisäurediester, 2-Propylheptyl-methyl-i-hexandisäurediester, 2-Propylheptyl-methyl-n-heptandisäurediester, 2-Propylheptyl-methyl-i-heptandisäurediester, 2-Propylheptyl-methyl-n-octandisäurediester, 2-Propylheptyl-methyl-i-octandisäurediester, 2-Propylheptyl-methyl-n-nonandisäurediester, 2-Propylheptyl-methyl-i-nonandisäurediester, 2-Propylheptyl-methyl-n-decandisäurediester, 2-Propylheptyl-methyl-i-decandisäurediester, 2-Propylheptyl-methyl-n-undecandi-säurediester, 2-Propylheptyl-methyl-i-undecandisäurediester, 2-Propylheptyl-methyl-n-undecendi-säurediester, 2-Propylheptyl-methyl-i-undecendisäurediester, 2-Propylheptyl-methyl-n-dodecandisäurediester, 2-Propylheptyl-methyl-i-dodecandisäurediester.

### Komponenten (b-2) Ester von 2-Ethylbutanol

Erfindungsgemäß als Komponenten (B) einsetzbar sind (b-2) Ester des **2-Ethylbutanols** mit linearen oder verzweigten, gesättigten oder ungesättigten C4-C36-Carbonsäuren - oder C4-C36-Dicarbonsäuren.

Diese Verbindungen sind in der unveröffentlichten Anmeldung EP 06017218.6 beschrieben, auf die hiermit ausdrücklich Bezug genommen wird.

In einer bevorzugten Ausführungsform der Erfindung werden Ester des 2-Ethylbutanols verwendet deren Gesamt C Zahl kleiner gleich 24, bevorzugt kleiner gleich 22 beträgt.

Erfindungsgemäß bevorzugt sind Estern des 2-Ethylbutanols mit Carbonsäuren, die ausgewählt sind aus den C4 bis C30, insbesondere C6 bis C24, insbesondere C6 bis C22, insbesondere C6 bis C18, insbesondere C8 bis C18 bevorzugt C8 bis C16, vorzugsweise C8 bis C12, insbesondere C6 bis C10 Carbonsäuren oder den entsprechenden Dicarbonsäuren.

Erfindungsgemäß eignen sich Ester des 2-Ethylbutanols mit C4 bis C36, C5 bis C30, C6 bis C26, C7 bis C24, C8 bis C22, C9 bis C20, C10 bis C18, C 11 bis C17, C11 bis C16, C12 bis C15, C13 bis C14 Carbonsäuren oder den entsprechenden Dicarbonsäuren.

In einer besonders bevorzugten Ausführungsform der Erfindung werden Ester des 2-Ethylbutanols mit Carbonsäuren, die ausgewählt sind aus den C6 bis C12 Carbonsäure sowie Estern des 2-Ethylbutanols mit Dicarbonsäuren, die ausgewählt sind aus den C6 bis C12 Dicarbonsäuren, eingesetzt.

Estern des 2-Ethylbutanols mit gesättigten Carbonsäuren sind erfindungsgemäß bevorzugt. Ester des 2-Ethylbutanols mit gesättigten Dicarbonsäuren sind erfindungsgemäß bevorzugt. Ester des 2-Ethylbutanols mit linearen, unverzweigten Carbonsäuren sind erfindungsgemäß bevorzugt. Ester des 2-Ethylbutanols mit linearen, unverzweigten Dicarbonsäuren sind erfindungsgemäß bevorzugt.

Bevorzugt sind die folgenden Ester: 2-Ethylbutyl-n-nonansäureester, 2-Ethylbutyl-i-nonansäureester, 2-Ethylbutyl-n-decansäureester, 2-Ethylbutyl-i-decansäureester, 2-Ethylbutyl-n-undecansäureester, 2-Ethylbutyl-i-undecansäureester, 2-Ethylbutyl-n-undecensäureester, 2-Ethylbutyl-n-dodecansäureester, 2-Ethylbutyl-i-dodecansäureester, 2-Ethylbutyl-n-tridecansäureester, 2-Ethylbutyl-i-tridecansäureester, 2-Ethylbutyl-n-tetradecansäureester, 2-Ethylbutyl-i-tetradecansäureester, 2-Ethylbutyl-n-pentadecansäureester, 2-Ethylbutyl-i-pentadecansäureester, 2-Ethylbutyl-n-hexadecansäureester, 2-Ethylbutyl-i-hexadecansäureester.

Besonders bevorzugt die folgenden Ester: 2-Ethylbutyl-n-nonansäureester, 2-Ethylbutyl-i-nonansäureester, 2-Ethylbutyl-n-decansäureester, 2-Ethylbutyl-i-decansäureester, 2-Ethylbutyl-n-dodecansäureester, 2-Ethylbutyl-i-dodecansäureester.

Der Begriff "Ester von 2-Ethylbutanol mit Dicarbonsäuren" umfasst sowohl Diester der Dicarbonsäuren mit 2-Ethylbutanol, also beispielsweise Di-2-Ethylbutyl-n-octandisäurediester als auch Monoester, wie z.B. 2-Ethylbutyl-n-octandisäuremonoester als auch gemischte Ester, in denen eine Säuregruppe der Dicarbonsäure mit 2-Ethylbutanol verestert ist und die zweite Säuregruppe der Dicarbonsäure mit einem weiteren Alkohol verestert ist. Eine weitere Ausführungsform der Erfindung umfasst gemischte Ester von Dicarbonsäuren und 2-Ethylbutanol und einem weiteren Alkohol der allgemeinen Formel R-OH, wobei R für einen linearen oder verzweigten, gesättigten oder ungesättigten Alkylrest mit 1 bis 12 C-Atomen steht.

In einer weiteren Ausführungsform werden eingesetzt gemischte Ester von Dicarbonsäuren und 2-Ethylbutanol und einem weiteren Alkohol der allgemeinen Formel R-OH, wobei R für einen gesättigten, linearen oder verzweigten, Alkylrest mit 1 bis 12 C-Atomen steht.

In einer bevorzugten Ausführungsform werden verwendet gemischte Ester von Dicarbonsäuren und 2-Ethylbutanol und einem weiteren Alkohol, wobei der weitere Alkohol ausgewählt ist aus der Gruppe bestehend aus Methanol, Ethanol, Propanol, Isopropanol Butanol, Isobutanol, Pentanol, Hexanol, Isohexanol, Octanol, Decanol oder Dodecanol.
In einer bevorzugten Ausführungsform der Erfindung werden Ester von 2-Ethylbutanol mit C4 bis C36 Dicarbonsäuren Diester und gemischte Ester verwendet.

Ein weiterer Gegenstand der Erfindung sind **Ester von 2-Ethylbutanol mit Dicarbonsäuren** ausgewählt aus linearen, verzweigten, gesättigten oder ungesättigten **C4 bis C36** Dicarbonsäuren. Die Erfindung umfasst sowohl einzelne Ester aus auch Mischungen von verschiedenen Estern.

Ester des 2-Ethylbutanols mit C9 bis C 16 Dicarbonsäuren sind erfindungsgemäß bevorzugt. Ester des 2-Ethylbutanols mit gesättigten Dicarbonsäuren sind erfindungsgemäß bevorzugt. Ester von 2-Ethylbutanols mit linearen, gesättigten Dicarbonsäuren sind erfindungsgemäß bevorzugt.

### Komponente (b-3) Ester von 2-Butyloctanol

Erfindungsgemäß als Komponenten (B) einsetzbar sind (b-3) (b-3) Estern des **2-Butyl-1-octanols** mit linearen oder verzweigten, gesättigten oder ungesättigten C2-C36-Carbonsäuren - oder C2-C36-Dicarbonsäuren

Diese Verbindungen sind in der deutschen Anmeldung EP 06017217.8 beschrieben, auf die hier ausdrücklich Bezug genommen wird.

Die Begriffe "2-Butyl-1-octanol" und "2-Butyloctanol" werden in der vorliegenden Beschreibung synonym verwendet, ebenso wie die Präfixe "2-Butyl-1-octyl" und "2-Butyloctyl"

In einer bevorzugten Ausführungsform der Erfindung werden Ester des 2-Butyloctanols verwendet deren Gesamt C Zahl kleiner gleich 24, bevorzugt kleiner gleich 22 beträgt.

Bevorzugt sind Ester des 2-Butyl-1-octanols mit Carbonsäuren, die ausgewählt sind aus den C4 bis C30, insbesondere C6 bis C24, insbesondere C6 bis C22, insbesondere C6 bis C18, insbesondere C8 bis C18 bevorzugt C8 bis C16, vorzugsweise C8 bis C12, insbesondere C6 bis C10 Carbonsäuren oder den entsprechenden Dicarbonsäuren.

Erfindungsgemäß eignen sich für Ester des 2-Butyl-1-octanols mit C2 bis C36, C5 bis C30, C6 bis C26, C7 bis C24, C8 bis C22, C9 bis C20, C10 bis C18, C11 bis C17, C11 bis C16, C12 bis C15, C13 bis C14 Carbonsäuren oder den entsprechenden Dicarbonsäuren.

In einer besonders bevorzugten Ausführungsform der Erfindung werden Estern des 2-Butyl-1-octanols mit Carbonsäuren, die ausgewählt sind aus den C4 bis C 16, C6 bis C12, C6 bis C10 Carbonsäuren sowie Estern des 2-Butyl-1-octanols mit Dicarbonsäuren, die ausgewählt sind aus den C4 bis C16, C6 bis C12, C6 bis C10 Dicarbonsäuren, eingesetzt.

Estern des 2-Butyl-1-octanols mit gesättigten Carbonsäuren sind erfindungsgemäß bevorzugt. Ester des 2-Butyl-1-octanols mit gesättigten Dicarbonsäuren sind erfindungsgemäß bevorzugt. Ester des 2-Butyl-1-octanols mit linearen, unverzweigten Carbonsäuren sind erfindungsgemäß bevorzugt. Estern des 2-Butyl-1-octanols mit linearen, unverzweigten Dicarbonsäuren ist erfindungsgemäß bevorzugt.

Exemplarisch seinen folgende Ester des 2-Butyl-1-octanols mit linearen oder verzweigten, gesättigten oder ungesättigten C2 bis C 36 Carbonsäuren genannt: 2-Butyloctylethansäureester, 2-Butyloctyl-propansäureester, 2-Butyloctyl-n-butansäureester, 2-Butyloctyl-i-butansäureester, 2-Butyioctyl-n-heptansäureester, 2-Butyloctyl-i-heptansäureester, 2-Butyloctyl-n-nonansäureester, 2-Butyloctyl-i-nonansäureester, 2-Butyloctyl-n-undecansäureester, 2-Butyloctyl-i-undecansäureester, 2-Butyloctyl-n-undecensäureester, 2-Butyloctyl-i-undecensäureester, 2-Butyloctyl-n-dodecansäureester, 2-Butyloctyl-i-dodecansäureester.

Der Begriff "Ester von 2-Butyl-1-octanols mit Dicarbonsäuren" umfasst sowohl Diester der Dicarbonsäuren mit 2-Butyl-1-octanols, also beispielsweise Di-2-butyloctyl-n-octandisäurediester als auch Monoester, wie z.B. 2-Butyl-octyl-n-octandisäuremonoester als auch gemischte Ester, in denen eine Säuregruppe der Dicarbonsäure mit 2-Butyl-1-octanol verestert ist und die zweite Säuregruppe der Dicarbonsäure mit einem weiteren Alkohol verestert ist. Eine weitere Ausführungsform der Erfindung umfasst gemischte Ester von Dicarbonsäuren und 2-Butyl-1-octanols und einem weiteren Alkohol der allgemeinen Formel R-OH, wobei R für einen linearen oder verzweigten, gesättigten oder ungesättigten Alkylrest mit 1 bis 12 C-Atomen steht.

In einer weiteren Ausführungsform werden eingesetzt gemischte Ester von Dicarbonsäuren und 2-Butyl-1-octanol und einem weiteren Alkohol der allgemeinen Formel R-OH, wobei R für einen gesättigten, linearen oder verzweigten, Alkylrest mit 1 bis 12 C-Atomen steht.

In einer bevorzugten Ausführungsform werden verwendet gemischte Ester von Dicarbonsäuren und 2-Butyl-1-octanol und einem weiteren Alkohol, wobei der weitere Alkohol ausgewählt ist aus der Gruppe bestehend aus Methanol, Ethanol, Propanol, Isopropanol Butanol, Isobutanol, Pentanol, Hexanol, Isohexanol, Octanol, Decanol oder Dodecanol.

In einer bevorzugten Ausführungsform der Erfindung werden Ester von 2-Butyl-1-octanols mit C2 bis C36 Dicarbonsäuren Diester und gemischte Ester verwendet.

Exemplarisch seinen folgende Ester des 2-Butyl-1-octanols mit linearen oder verzweigten, gesättigten oder ungesättigten C2 bis C 36 Dicarbonsäuren genannt: Di-2-Butyloctylethandisäurediester, Di-2-Butyloctyl-propandisäurediester, Di-2-Butyloctyl-n-butandisäurediester, Di-2-Butyloctyl-i-butandisäurediester, Di-2-Butyloctyl-n-heptandisäurediester, Di-2-Butyloctyl-i-heptandisäurediester, Di-2-Butyloctyl-n-nonandisäurediester, Di-2-Butyloctyl-i-nonandisäurediester, Di-2-Butyloctyl-n-undecandisäurediester, Di-2-Butyloctyl-i-undecandisäurediester, Di-2-Butyloctyl-n-undecendisäurediester, Di-2-Butyloctyl-i-undecendisäurediester, Di-2-Butyloctyl-n-dodecandisäurediester, Di-2-Butyloctyl-i-dodecandisäurediester.

### Beispiele

### Beispiel 1: Eine Ölkörper Mischung bestehend aus 90 Gew.-% n-Dodecan und 10 Gew.-% 2-Propylheptytoctanoat wurde durch Mischen der 2 Bestandteile hergestellt.

Der sensorische Vergleich dieser erfindungsgemäßen Mischung erfolgte gegen Cyclopentamethicone (DC 245, Fa. Dow Corning). Die erfindugsgemäße Ölkörper Mischung zeigte eine mit Cyclopentamethicone vergleichbare Sensorik.

## Patentansprüche

1. Ölkörper Mischung, enthaltend
(A) mindestens einen flüchtigen Kohlenwasserstoff und
(B) mindestens einen Ester ausgewählt aus der Gruppe bestehend aus
(b-1) Estern des **2-Propylheptanols** mit linearen oder verzweigten, gesättigten oder ungesättigten C4-C36-Carbonsäuren - oder C4-C36-Dicarbonsäuren,
(b-2) Estern des 2-Ethylbutanols mit linearen oder verzweigten, gesättigten oder ungesättigten C4-C36-Carbonsäuren - oder C4-C36-Dicarbonsäuren, und
(b-3) Estern des **2-Butyl-1-octanols** mit linearen oder verzweigten, gesättigten oder ungesättigten C2-C36-Carbonsäuren - oder C2-C36-Dicarbonsäuren
sowie Mischungen daraus.

2. Mischung nach Anspruch 1, **dadurch gekennzeichnet, dass** sie 60 Gew.-% oder mehr, insbesondere 70 Gew.-% oder mehr, insbesondere 80 Gew.-% oder mehr, besonders bevorzugt 90 Gew.-% oder mehr, insbesondere 95 Gew.-% oder mehr der Komponenten (A) und (B) enthält.

3. Mischung nach einem der vorgenannte Ansprüche, **dadurch gekennzeichnet, dass** die Menge an (A) größer gleich 50 Gew.-% bezogen auf die Gesamtmenge von (A) und (B) beträgt, insbesondere größer gleich 60 Gew.-%, bevorzugt größer gleich 70 Gew.-%.

4. Mischung nach einem der vorgenannten Ansprüche, **dadurch gekennzeichnet, dass** sie weniger als 5 Gew.-%, insbesondere weniger als 3 Gew.-%, insbesondere weniger als 1 Gew.-% Silikonöle enthält.

5. Mischung nach einem der vorgenannten Ansprüche, **dadurch gekennzeichnet, dass** sie keine Silikonöle enthält.

6. Kosmetische Zubereitung, enthaltend
(A) mindestens einen flüchtigen Kohlenwasserstoff und
(B) mindestens einen Ester ausgewählt aus der Gruppe bestehend aus
(b-1) Estern des **2-Propylheptanols** mit linearen oder verzweigten, gesättigten oder ungesättigten C4-C36-Carbonsäuren - oder C4-C36-Dicarbonsäuren,
(b-2) Estern des 2-Ethylbutanols mit linearen oder verzweigten, gesättigten oder ungesättigten C4-C36-Carbonsäuren - oder C4-C36-Dicarbonsäuren, und
(b-3) Estern des **2-Butyl-1-octanols** mit linearen oder verzweigten, gesättigten oder ungesättigten C2-C36-Carbonsäuren - oder C2-C36-Dicarbonsäuren
sowie Mischungen daraus.

7. Kosmetische Zubereitungen nach Anspruch 6, enthaltend weniger als 5 Gew.-%, insbesondere weniger als 3 Gew.-%, insbesondere weniger als 1 Gew.-% Silikonöle.

8. Kosmetische Zubereitungen nach Anspruche 6 oder 7, **dadurch gekennzeichnet, dass** sie keine Silikonöle enthalten.

9. Kosmetische Zubreitung, nach mindestens einem der Ansprüche 6 bis 8, **dadurch gekennzeichnet, dass** die kosmetische Zubereitung neben (A) und (B) weniger als 40 Gew.-%, insbesondere weniger als 30 Gew.-%, insbesondere weniger als 20 Gew.-%, bevorzugt weniger als 10 Gew.-% weiterer lipophiler Komponenten enthält.

10. Verwendung von Ölkörper Mischungen nach mindestens einem der Ansprüche 1 bis 5 in kosmetischen Zubereitungen.

11. Verwendung von Ölkörper Mischungen nach mindestens einem der Ansprüche 1 bis 5 als Silikonersatz in kosmetischen Zubereitungen.

12. Mischung, Zubereitung oder Verwendung nach einem der vorgenannten Ansprüche, **dadurch gekennzeichnet, dass** als flüchtiger Kohlenwasserstoff (A) gesättigte und ungesättigte , lineare und verzweigte, cyclische Kohlenwasserstoffe mit einer C Zahl von 6 bis 20 eingesetzt werden.

13. Mischung, Zubereitung oder Verwendung nach einem der vorgenannten Ansprüche, **dadurch gekennzeichnet, dass** als flüchtige Kohlenwasserstoffe Mineralöle eingesetzt werden.
